# EUROPÄISCHE PATENTSCHRIFT

(11) **EP 2 233 086 B1**
(45) Veröffentlichungstag und Bekanntmachung des Hinweises auf die Patenterteilung: **01.06.2011**
(21) Anmeldenummer: 10152418.9
(22) Anmeldetag: 02.02.2010
(51) Int. Cl.: A61B 17/30

(54) **Vorrichtung zum Entfernen von Parasiten**
Tick removing device
Dispositif pour extraire les tiques

(30) Priorität: 19.02.2009 DE 102009009736
(43) Veröffentlichungstag der Anmeldung: 29.09.2010
(73) Patentinhaber: Karagün, Ahmed, 89362 Offingen (DE)
(72) Erfinder: Karagün, Ahmed, 89362 Offingen (DE)
(74) Vertreter: Schwarz, Thomas

(56) Entgegenhaltungen:
- EP-A1- 0 811 355
- DE-A1- 10 023 212
- US-A- 5 407 243
- US-A- 5 914 062
- US-A1- 2007 185 528

## Beschreibung

Die Erfindung betrifft eine Vorrichtung zum Entfernen von Parasiten, insbesondere Zecken, nach dem Oberbegriff des Anspruchs 1.

Aus der DE 10 2004 031 682 A1 ist eine gattungsgemäße Vorrichtung zum Entfernen von Zecken bekannt, bei der innerhalb eines stiftförmigen Gehäuses eine Greifeinrichtung mit einem spreizbaren Greifwerkzeug angeordnet ist. In dem Gehäuse befindet sich außerdem eine als Druckstift ausgeführte Druckvorrichtung, die auf eine gesonderte Spreizeinrichtung zum Spreizen des Greifwerkzeugs und eine Dreheinrichtung zum Drehen des Greifwerkzeugs wirkt. Zwischen dem Druckstift und dem Gehäuse ist eine im Inneren des Gehäuses angeordnete Druckfeder eingespannt. Diese bekannte Vorrichtung ist so konzipiert, dass eine erste Betätigung der Druckvorrichtung eine Spreizung des Greifwerkzeugs und eine weitere Betätigung eine Drehung des Greifwerkzeugs bewirkt. Das Greifwerkzeug ist derart ausgestaltet, dass es in seinem ungespreizten Zustand einen im Wesentlichen geschlossenen Hohlraum zur Aufnahme der Zecke umschließt. Durch den zur Realisierung der kombinierten Längs- und Drehbewegung erforderlichen Gewindeantrieb weist diese bekannte Vorrichtung jedoch einen entsprechend erhöhten Fertigungsaufwand auf. Außerdem wird eine vergleichsweise aufwändige Spreizeinrichtung zum Spreizen der einen Hohlraum bildenden Greifwerkzeuge benötigt.

Aufgabe der Erfindung ist es, eine Vorrichtung der eingangs genannten Art zu schaffen, die auf kostengünstige Weise eine vereinfachte und sichere Entfernung von Parasiten ermöglicht.

Diese Aufgabe wird durch eine Vorrichtung mit den Merkmalen des Anspruchs 1 gelöst. Vorteilhafte Ausgestaltungen und zweckmäßige Weiterbildungen der erfindungsgemäßen Vorrichtung sind Gegenstand der Unteransprüche.

Bei der erfindungsgemäßen Vorrichtung enthält die innerhalb eines Gehäuses axial verschiebbar angeordnete Greiferzange zwei gegeneinander bewegliche Greiferarme und ein manuell betätigbares Druckelement, das durch eine erste Druckfeder beaufschlagt und zur Bewegung der Greiferarme zwischen einer Schließstellung und einer Offenstellung bewegbar ist. Die Greiferzange ist innerhalb eines im Gehäuse verschiebbaren und durch eine zweite Druckfeder beaufschlagten Innenteils verschiebbar geführt. Durch die zweite Druckfeder kann die Greiferzange auch dann weiterhin unter Zug gehalten werden, wenn die Greiferarme vollständig geschlossen sind. Der durch die Greiferzange erfasste Parasit kann durch Drehung des Gehäuses unter Zugwirkung der zweiten Druckfeder gedreht werden, wodurch der Parasit einfach und sicher aus der Haut entfernt werden kann. Dadurch dass die Greiferzange bei der Drehung unter Zugspannung gehalten wird, kann ein Abreißen des Kopfs der Parasiten vermieden werden.

In einer besonders vorteilhaften und zweckmäßigen Ausgestaltung der Erfindung ist das im Bereich der Greiferarme angeordnete Vorderteil des Gehäuses aus einem durchsichtigen Material mit einer Vergrößerungsfunktion in Art einer Lupe ausgeführt. Dadurch kann das korrekte Ansetzen der Vorrichtung besser beobachtet werden. Außerdem kann nach dem Entfernen des Parasiten einfacher kontrolliert werden, ob dieser auch vollständig mit allen Teilen entfernt wurde.

Bei einer konstruktiv einfachen Ausführung ist die erste Druckfeder zwischen einer inneren ringförmigen Anlagefläche des Innenteils und einem an Ende des Druckelements befestigten Druckstück eingespannt. Die zweite Druckfeder ist dagegen zwischen einem inneren Ringbund des Gehäuses und einem äußeren Ringbund des Innenteils eingespannt.

Weitere Besonderheiten und Vorteile der Erfindung ergeben sich aus der folgenden Beschreibung eines bevorzugten Ausführungsbeispiels anhand der Zeichnung. Es zeigen:
- **Figur 1:**: eine Seitenansicht einer erfindungsgemäßen Vorrichtung zum Entfernen von Parasiten;
- **Figur 2:**: eine Schnittansicht entlang der Linie D-D von Figur 1;
- **Figur 3:**: einen Längsschnitt der Vorrichtung von Figur 1 in der Ausgangsstellung;
- **Figur 4:**: einen Längsschnitt der Vorrichtung von Figur 1 in einer ersten Ausfahrstellung und
- **Figur 5:**: einen Längsschnitt der Vorrichtung von Figur 1 in einer geöffneten Stellung.

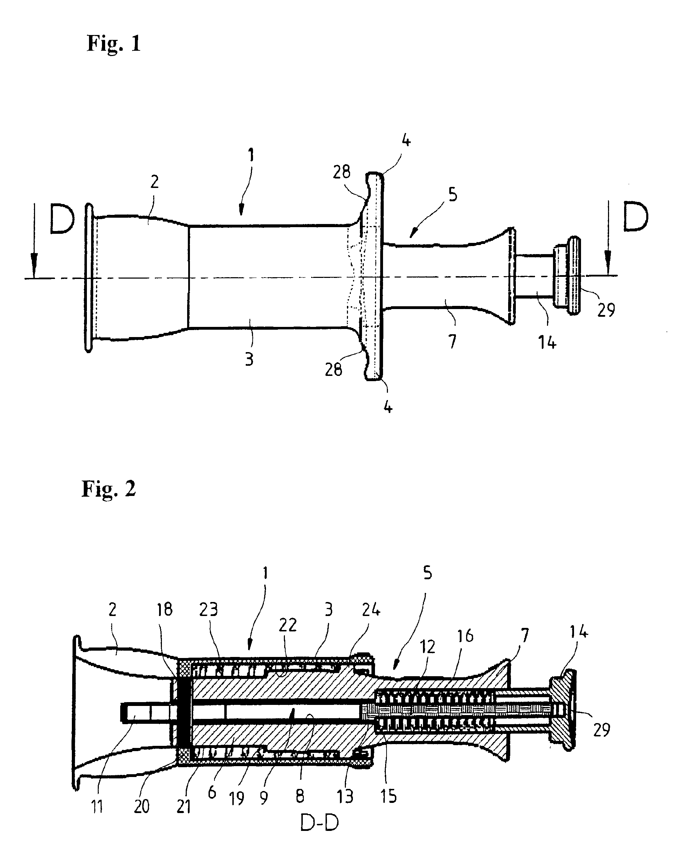

Die in den Figuren 1 bis 5 in verschiedenen Stellungen und Ansichten gezeigte Vorrichtung zur Entfernung von Parasiten, insbesondere Zecken, enthält ein Gehäuse 1, das ein Vorderteil 2 und ein hinteres Endstück 3 mit zwei diametral gegenüberliegenden, seitlich vorstehenden Griffstücken 4 enthält. Das Vorderteil 2 des Gehäuses 1 ist aus einem durchsichtigen Material hergestellt und weist eine Vergrößerungsfunktion in Art einer Lupe bzw. eines Vergrößerungsglases auf. Dadurch kann der zu entfernende Parasit beim Aufsetzen der Vorrichtung genau betrachtet und die Positionierung der Vorrichtung kontrolliert werden. Außerdem kann nach dem Herausziehen des Parasiten einfacher überprüft werden, ob der Parasit mit allen seinen Teilen vollständig entfernt worden ist. Die Vergrößerungsfunktion kann z.B. dadurch erreicht werden, dass das durchsichtige Vorderteil 2 mehrere über den Umfang verteilte linsenförmige Bereiche oder eine linsenförmige Wölbung aufweist.

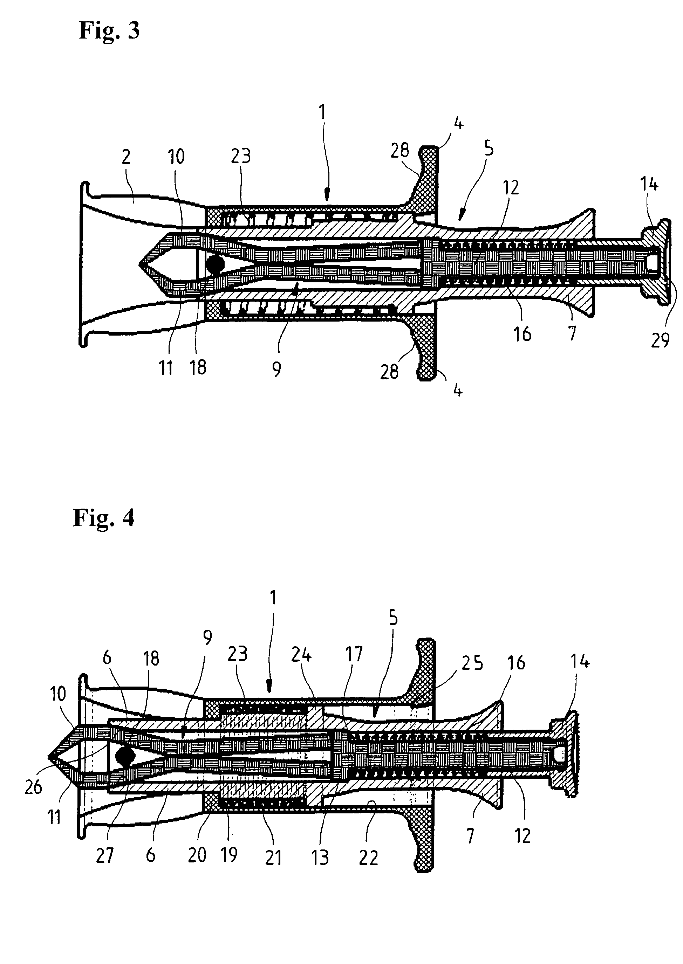

In dem als Hohlkörper ausgeführten Gehäuse 1 ist ein im Wesentlichen hohlzylindrisches Innenteil 5 zwischen einer in den Figuren 1 und 2 gezeigten hinteren Ausgangsstellung und einer in den Figuren 3 bis 6 gezeigten vorderen Eindrückstellung axial verschiebbar geführt. Das Innenteil 5 weist einen innerhalb des Gehäuses 1 angeordneten vorderen Bereich 6 und einen gegenüber dem Gehäuse 1 nach hinten vorstehenden, sich konisch erweitenden hinteren Bereich 7 auf.

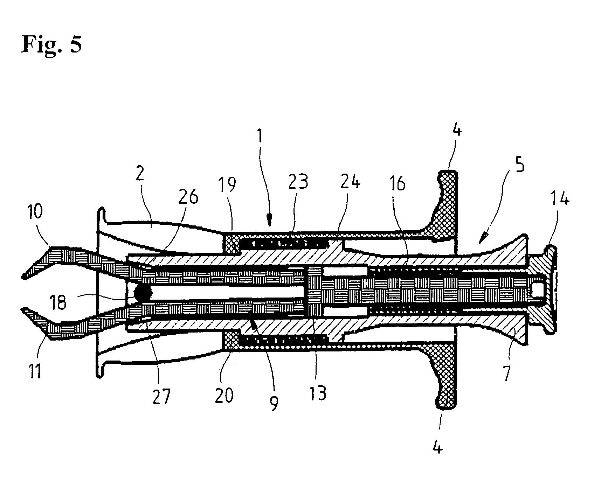

In einer Durchgangsöffnung 8 des Innenteils 5 ist eine Greiferzange 9 mit zwei zwischen einer in den Figuren 3 und 4 gezeigten Schließstellung und einer in den Figuren 5 dargestellten Offenstellung beweglichen Greiferarmen 10 bzw. und 11 axial verschiebbar geführt. Die Greiferzange 9 enthält ein gegenüber dem Innenteil 5 nach hinten vorstehendes, in Querschnitt rechteckiges Druckelement 12 und einen in der Durchgangsöffnung 8 verschiebbar geführten Absatz 13, an dem die in Art einer Pinzette ausgeführten, nach vorne vorstehenden Greiferarme 10 und 11 angeformt sind. An dem hinteren Ende des stangenförmigen Druckelements 12 ist ein hinteres Druckstück 14 befestigt. Wie aus Figur 2 hervorgeht, ist zwischen einer inneren ringförmigen Anlagefläche 15 des Innenteils 5 und dem hinteren Druckstück 14 eine um das stangenförmige Druckelement 12 angeordnete erste Druckfeder 16 eingespannt. Der im Querschnitt ebenfalls rechteckige Absatz 13 liegt in der Schließstellung der Greiferzange 16 an einem in Figur 4 erkennbaren Ansatz 17 des Innenteils 5 an. Zwischen den beiden Greiferarmen 10 und 11 ist ein Querstift 18 derart angeordnet, dass beim Eindrücken der Greiferzange 9 die beiden Greiferzangen 10 und 11 auseinander bewegt werden.

Wie aus Figur 2 erkennbar ist, enthält das Innenteil 5 an der Außenseite des vorderen Bereichs 6 einen Absatz 19, der bei der in Figur 4 gezeigten vorderen Eindrückstellung zur Anlage an einem inneren Ringbund 20 des Gehäuse 1 gelangt. Zwischen einer Außenseite 21 des Innenteils 5 und einer Innenwand 22 des Gehäuses 1 ist eine zweite Druckfeder 23 angeordnet, die sich mit dem vorderen Ende an dem inneren Ringbund 20 des Gehäuses 1 und mit dem hinteren Ende auf einem äußeren Ringbund 24 des Innenteils 5 abstützt. Im hinteren Bereich des Gehäuses 1 ist an der Innenwand 22 ein in Figur 4 erkennbarer Ansatz 25 vorgesehen, an dem der äußere Ringbund 24 bei der in Figur 1 gezeigten Ausgangsstellung zur Anlage gelangt. Das innerhalb des Gehäuses 1 verschiebbar geführte Innenteil 5 kann so entgegen der Kraft der zweiten Druckfeder 23 nach vorne gedrückt werden und wird bei nachlassendem Druck durch die Druckfeder 23 wieder nach hinten bewegt, bis der äußere Ringbund 24 an dem Ansatz 25 anliegt.

Wie aus Figur 4 hervorgeht, liegen die beiden Greiferarme 10 und 11 in der dort gezeigten Schließstellung mit ihren Außenseiten 26 an schrägen Innenflächen 27 im vorderen Teil der Durchgangsöffnung 15 des Innenteils 5 an und werden dadurch zusammengedrückt. Wenn die Greiferzange 9 jedoch relativ zum Innenteil 5 entgegen der ersten Druckfeder 16 nach vorne gedrückt wird, gelangen die beiden Greiferarme 10 und 11 außer Eingriff mit den Innenflächen 27 und werden durch den Querstift 18 zum Öffnen der Greiferzange 9 auseinandergedrückt, wie dies aus Figur 5 hervorgeht.

Zum Entfernen eines Parasiten aus der Haut wird die vorstehend beschriebene und in der Zeichnung dargestellte Vorrichtung zwischen Daumen und dem Zeige- und Mittelfinger gehalten, wobei der Zeige- und Mittelfinger zur Anlage an in Figur 3 gezeigten vorderen Griffflächen 28 der beiden seitlich vorstehenden Griffstücke 4 gelangen und der Daumen auf eine hintere Druckfläche 29 des Druckstücks 14 drückt. Durch Eindrücken des Druckstücks 14 mit Hilfe des Daumens wird gemäß Figur 4 zunächst das Innenteil 5 relativ zum äußeren Gehäuse 1 entgegen der Kraft der zweiten Druckfeder 23 nach vorne geschoben, wobei die beiden Greiferarme 10 und 11 der Greiferzange 9 noch geschlossen bleiben. Erst in einer nächsten Stufe wird auch das Druckelement 12 entgegen der Kraft der ersten Duckfeder 16 relativ zum Innenteil 5 nach vorne verschoben, wobei sich die beiden Greiferarme 10 und 11 gemäß Figur 5 öffnen.

In der geöffneten Stellung von Figur 5 kann die Vorrichtung z.B. über eine auf Haut befindliche Zecke aufgesetzt werden. Bei nachlassendem Druck an dem hinteren Druckstück 14 des Druckelements 12 fahren zunächst die beiden Greiferarme 10 und 11 zusammen, wodurch die Zecke gegriffen werden kann. Über das als Lupe ausgebildete Vorderteil 2 des Gehäuses 1 kann dabei das korrekte und vollständige Greifen des Parasiten überprüft werden. Bei weiter nachlassendem Druck am hinteren Druckstück 14 des Druckelements 12 fährt das Innenteil 5 unter der Wirkung der zweiten Druckfeder 23 ein, wobei auch in dieser Phase die Greiferzange 9 unter Zug steht. Die gesamte Vorrichtung kann dabei auch leicht gedreht werden, wodurch die vollständige Entfernung z.B. einer Zecke erleichtert wird. Auch wenn die Zecke durch die beiden Greiferarme 10 und 11 in das Gehäuse 1 eingezogen ist, kann über das als Lupe bzw. Vergrößerungsglas ausgeführte Vorderteil 2 überprüft werden, ob die Zecke mit sämtlichen Teilen entfernt wurde.

## Patentansprüche

1. Vorrichtung zum Entfernen von Parasiten mit einem Gehäuse (1) und einer innerhalb des Gehäuses (1) axial verschiebbar angeordneten Greiferzange (9), die zwei gegeneinander bewegliche Greiferarme (10, 11) und ein manuell betätigbares und durch eine erste Druckfeder (16) beaufschlagtes Druckelement (12) zur Bewegung der Greiferarme (10, 11) zwischen einer Offenstellung und einer Schließstellung zum Greifen des Parasiten enthält, **dadurch gekennzeichnet, dass** die Greiferzange (9) innerhalb eines im Gehäuse (1) verschiebbaren und durch eine zweite Druckfeder (23) beaufschlagten Innenteils (5) verschiebbar geführt ist.

2. Vorrichtung nach Anspruch 1, **dadurch gekennzeichnet, dass** ein im Bereich der Greiferarme (10, 11) angeordnetes Vorderteil (2) des Gehäuses (1) aus einem durchsichtigen Material mit einer Vergrößerungsfunktion besteht.

3. Vorrichtung nach Anspruch 1 oder 2, **dadurch gekennzeichnet, dass** die erste Druckfeder (16) zwischen einer inneren ringförmigen Anlagefläche (15) des Innenteils (5) und einem an Ende des Druckelements (12) befestigten Druckstück (14) eingespannt ist.

4. Vorrichtung nach einem der Ansprüche 1 bis 3, **dadurch gekennzeichnet, dass** die zweite Druckfeder (23) zwischen einem inneren Ringbund (20) des Gehäuses (1) und einem äußeren Ringbund (24) des Innenteils (5) eingespannt ist.

5. Vorrichtung nach einem der Ansprüche 1 bis 4, **dadurch gekennzeichnet, dass** zwischen den beiden Greiferarmen (10, 11) ein Querstift (18) derart angeordnet ist, dass beim Eindrücken der Greiferzange (9) die beiden Greiferarme (10, 11) auseinander bewegt werden.

6. Vorrichtung nach einem der Ansprüche 1 bis 5, **dadurch gekennzeichnet, dass** die Greiferzange (9) mit den beiden gegeneinander beweglichen Greiferarmen (10, 11) in einer Durchgangsöffnung (8) des Innenteils (5) axial verschiebbar geführt ist.

7. Vorrichtung nach einem der Ansprüche 1 bis 6, **dadurch gekennzeichnet, dass** das Innenteil (5) in dem als Hohlkörper ausgebildeten Gehäuse (1) zwischen einer hinteren Ausgangsstellung und einer vorderen Eindrückstellung axial verschiebbar geführt ist.

8. Vorrichtung nach einem der Ansprüche 1 bis 7, **dadurch gekennzeichnet, dass** das Gehäuse (1) ein hinteres Endstück (3) mit zwei diametral gegenüberliegenden, seitlich vorstehenden Griffstücken (4) enthält.

9. Vorrichtung nach einem der Ansprüche 1 bis 8, **dadurch gekennzeichnet, dass** das Gehäuse (1) einen hinteren Ansatz (25) zur Anlage des Innenteils (5) in einer hinteren Ausgangsstellung aufweist.

10. Vorrichtung nach einem der Ansprüche 1 bis 9, **dadurch gekennzeichnet, dass** am hinteren Ende des Druckelements (12) ein Druckstück (14) befestigt ist.

## Claims

1. Device for removing parasites with a housing (1) and gripping forceps (9) which are arranged displaceable axially inside the housing (1) and comprise two gripping arms (10, 11) which can be moved against one another, and a thrust element (12) which can be operated manually and is subject to the action of a first compression spring (16) for moving the gripping arms (10, 11) between an open position and a closed position to grip the parasite, **characterised in that** the gripping forceps (9) are guided displaceably inside an internal part (5) which is displaceable in the housing (1) and subject to the action of a second compression spring (23).

2. Device according to claim 1, **characterised in that** a front part (2) of the housing (1) arranged in the area of the gripping arms (10, 11) is made of a transparent material with a magnifying function.

3. Device according to claim 1 or 2, **characterised in that** the first compression spring (16) is clamped between an inner annular bearing face (15) of the internal part (5) and a thrust piece (14) fastened to the end of the thrust element (12).

4. Device according to one of claims 1 to 3, **characterised in that** the second compression spring (23) is clamped between an inner annular collar (20) of the housing (1) and an outer annular collar (24) of the internal part (5).

5. Device according to one of claims 1 to 4, **characterised in that** a transverse pin (18) is arranged between the two gripping arms (10, 11) in such a way that when the gripping forceps (9) are pressed in, the two gripping arms (10, 11) are moved away from one another.

6. Device according to one of claims 1 to 5, **characterised in that** the gripping forceps (9) with the two gripping arms (10, 11) which can be moved against one another are guided displaceable axially in an opening (8) running through the internal part (5).

7. Device according to one of claims 1 to 6, **characterised in that** the internal part (5) is guided displaceable axially in the housing (1) in the form of a hollow body between a rear starting position and a front impressing position.

8. Device according to one of claims 1 to 7, **characterised in that** the housing (1) comprises a rear end piece (3) with two diametrically opposing, laterally projecting gripping pieces (4).

9. Device according to one of claims 1 to 8, **characterised in that** the housing (1) exhibits a rear shoulder (25) for the internal part (5) to bear on in a rear starting position.

10. Device according to one of claims 1 to 9, **characterised in that** a thrust piece (14) is fastened to the rear end of the thrust element (12).

## Revendications

1. Dispositif pour extraire des parasites doté d'un boîtier (1) et d'une pince (9) disposée de manière mobile dans le sens axial dans le boîtier (1), qui contient deux bras de pince (10, 11) mobiles l'un contre l'autre et un élément de compression (12) pouvant être actionné manuellement et sollicité par un premier ressort de compression (16) pour le déplacement des bras de pince (10, 11) entre une position ouverte et une position fermée destinée à la préhension du parasite, **caractérisé en ce que** la pince (9) est guidée de manière mobile dans une partie intérieure (5) mobile dans le boîtier (1) et sollicitée par un second ressort de compression (23).

2. Dispositif selon la revendication 1, **caractérisé en ce qu'**une partie avant (2) du boîtier (1) disposée dans la zone des bras de pince (10, 11) se compose d'un matériau transparent présentant une fonction d'agrandissement.

3. Dispositif selon la revendication 1 ou 2, **caractérisé en ce que** le premier ressort de compression (16) est serré entre une surface d'appui (15) intérieure annulaire de la partie intérieure (5) et une pièce de compression (14) fixée sur l'extrémité de l'élément de compression (12).

4. Dispositif selon l'une quelconque des revendications 1 à 3, **caractérisé en ce que** le second ressort de compression (23) est serré entre un collet annulaire intérieur (20) du boîtier (1) et un collet annulaire extérieur (24) de la partie intérieure (5).

5. Dispositif selon l'une quelconque des revendications 1 à 4, **caractérisé en ce qu'**une goupille transversale (18) est disposée entre les deux bras de pince (10, 11) de telle manière que lors du pressage de la pince (9), les deux bras de pince (10, 11) sont écartés l'un de l'autre.

6. Dispositif selon l'une quelconque des revendications 1 à 5, **caractérisé en ce que** la pince (9) est guidée de manière mobile dans le sens axial avec les deux bras de pince (10, 11) mobiles l'un contre l'autre dans une ouverture débouchante (8) de la partie intérieure (5).

7. Dispositif selon l'une quelconque des revendications 1 à 6, **caractérisé en ce que** la partie intérieure (5) est guidée de manière mobile dans le sens axial dans le boîtier (1) réalisé comme un corps creux entre une position de départ arrière et une position de pressage avant.

8. Dispositif selon l'une quelconque des revendications 1 à 7, **caractérisé en ce que** le boîtier (1) contient une pièce d'extrémité arrière (3) avec deux poignées (4) faisant saillie latéralement, diamétralement opposées.

9. Dispositif selon l'une quelconque des revendications 1 à 8, **caractérisé en ce que** le boîtier (1) présente une saillie arrière (25) pour l'appui de la partie intérieure (5) dans une position de départ arrière.

10. Dispositif selon l'une quelconque des revendications 1 à 9, **caractérisé en ce qu'**une pièce de compression (14) est fixée sur l'extrémité arrière de l'élément de compression (12).
